# EUROPEAN PATENT SPECIFICATION

(11) **EP 4 294 810 B1**
(45) Date of publication and mention of the grant of the patent: **16.10.2024**
(21) Application number: 22712444.3
(22) Date of filing: 18.02.2022
(51) Int. Cl.: C07D 487/04, A61K 31/519

(54) **PROCESS FOR PREPARING HIGH PURITY DEGREE LAROTRECTINIB**
VERFAHREN ZUR HERSTELLUNG VON HOCHREINEM LAROTRECTINIB
PROCÉDÉ DE PRÉPARATION DE LAROTRECTINIB DE PURETÉ ÉLEVÉE

(30) Priority: 19.02.2021 IT 202100003887
(43) Date of publication of application: 27.12.2023
(73) Proprietor: OLON S.p.A., 20053 Rodano (MI) (IT)
(72) Inventor: TRABACE, Simona, 20053 Rodano (MI) (IT); DE FIORE, Stella, 20053 Rodano (MI) (IT); FUSARI, Matteo, 20053 Rodano (MI) (IT); BONANOMI, Jacopo, 20053 Rodano (MI) (IT); NOVO, Barbara, 20053 Rodano (MI) (IT); SADA, Mara, 20054 Segrate (MI) (IT); BERTOLINI, Giorgio, 20053 Rodano (MI) (IT)
(74) Representative: Bottero, Carlo
(86) International application number: PCT/IB2022/051461
(87) International publication number: WO 2022/175883

(56) References cited:
- WO-A1-2016/077841
- WO-A1-2019/220352
- SCOTT LESLEY J.: "Larotrectinib: First Global Approval", vol. 79, no. 2, 1 February 2019 (2019-02-01), NZ, pages 201 - 206, XP055843384, ISSN: 0012-6667, Retrieved from the Internet <URL:http://link.springer.com/content/pdf/10.1007/s40265-018-1044-x.pdf> DOI: 10.1007/s40265-018-1044-x

## Description

The present invention relates to a Larotrectinib synthesis process with a 3-O-sulfonylated impurity content lower than 0.10% by weight. A further object of the invention is the compound 3-O-sulfo-Larotrectinib ((3S)-N-[5-[(2R)-2-(2,5-Difluorophenyl)-1-pyrrolidinyl]pyrazole[1,5-a]pyrimidine-3-yl]-3-hydroxysulphonyloxy-1-pyrrolidinecarboxamide), hereinafter referred to as S-Larotrectinib, useful as an analytical standard to evaluate the purity of Larotrectinib.

### State of the art

Larotrectinib ((3 S)-N-[5-[(2R)-2-(2,5-Difluorophenyl)-1-pyrrolidinyl]pyrazole-[1,5-a]pyrimidine-3-yl]-3-hydroxy-1-pyrrolidinecarboxamide) of formula I: 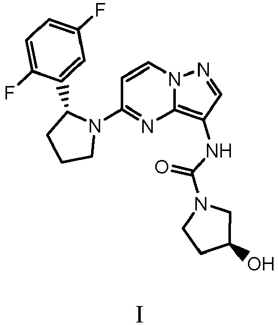 is a drug (trade name Vitravki^{®}), indicated in the treatment of solid tumors characterized by an NTRK gene fusion in adult and pediatric patients with locally advanced disease, metastatic disease or when other acceptable therapeutic options are present. Larotrectinib is marketed in capsules or oral solution, in the form of sulfate salt.

Any impurities deriving from the synthesis process or from degradative processes must be carefully identified and monitored in order to guarantee appropriate safety and quality standards, as required, for examples, by international guidelines such as ICH Q7, a guideline concerning good laboratory practices (GMP) which provides the parameters to manage an appropriate purity control system of the active ingredient and the ICH Q3 A, which indicates the limits below which impurities and residual solvents must be maintained.

By reproducing the synthesis of Larotrectinib described in WO2016077841 (example 2, page 66), the only example reported with quantities applicable on an industrial scale, the API is obtained in the form of sulphate salt with a purity close to the limits of the ICH guidelines and which is difficult to control.

In the process described in WO2016077841, Larotrectinib sulfate is precipitated with the addition of an anti-solvent from an ethanol solution. This step on an industrial scale is very delicate and leads to the formation of a precipitate that is difficult to manage. Said precipitate has a rubbery consistency which leads to problems in the processing phase, especially during filtration, and to the absorption of a large amount of ethanol, which must be reduced to 0.5% by weight with respect to the total mass in the subsequent stage of hot suspension in water and 2-butanone.

### Description of invention:

It has now been found that it is possible to obtain Larotrectinib of high purity by replacing ethanol with dichloromethane in the conversion from hydrochloride salt to sulphate salt.

A previously unidentified impurity corresponding to ((3S)-N-[5-[(2R)-2-(2,5-Difluorophenyl)-1-pyrrolidinyl]-pyrazole [1,5- a] pyrimidine-3-yl] -3-hydroxysulfonyloxy-1-pyrrolidinecarboxamide of formula II (S-Larotrectinib) was also isolated:

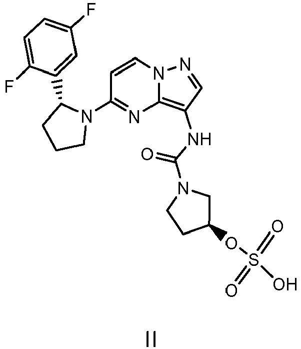

In a first aspect, the invention therefore provides a process for preparing Larotrectinib sulfate containing less than 0.10% by weight S-Larotrectinib, comprising the following steps:
(a) the unblocking reaction of Larotrectinib hydrochloride in dichloromethane, in the presence of bases;
(b) the treatment of Larotrectinib obtained in step a) with sulfuric acid in methyl ethyl ketone, and water;
(c) the filtration of Larotrectinib sulfate.

In a second aspect, an object of the invention relates to the compound ((3S)-N-[5-[(2R)-2-(2,5-Difluorophenyl)-1-pyrrolidinyl]pyrazole[1,5-a]pyrimidine-3-yl]-3-hydroxysulfonyloxy-1-pyrrolidinecarboxamide of formula II (S-Larotrectinib):

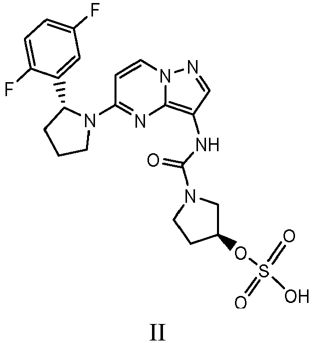

Another object of the invention is an analytical procedure for the determination and quantitation of S-Larotrectinib.

Larotrectinib chloride, synthesized according to known methods, is transformed into Larotrectinib sulfate by means of an unblocking reaction in the presence of dichloromethane, and of a base, selected from alkaline or alkaline earth hydroxides, in particular NaOH or KOH. Said reaction is carried out at room temperature. The formation of Larotrectinib sulfate can occur by dropping a 40% by weight sulfuric acid aqueous solution into a solution of demineralized water and methylethyl ketone 5:95 at room temperature.

The Larotrectinib sulphate thus obtained is filtered and dried at room temperature, to give without further processing the desired product within specification to the content of residual solvents and impurities in accordance to the ICH guidelines indicated above.

Another object of the invention is a process to determine the presence of S-Larotrectinib in a sample of Larotrectinib sulfate, comprising an HPLC analysis, which uses S-Larotrectinib compound as a reference standard.

Said process comprises:
a) the determination, by HPLC analysis, of the retention time corresponding to S-Larotrectinib using a reference sample of S-Larotrectinib;
b) the determination, by HPLC analysis, of the retention time corresponding to S-Larotrectinib in a sample of Larotrectinib sulphate;
c) the determination of the presence of S-Larotrectinib in a sample of Larotrectinib sulphate by comparing the chromatograms obtained in the steps a) and b).

Another object of the invention is a process to determine the amount of S-Larotrectinib in a sample of Larotrectinib sulfate, comprising an HPLC analysis using S-Larotrectinib as a reference standard.

Said process comprises:
a) the calculation by HPLC of the area corresponding to S-Larotrectinib in the reference standard characterized by a known amount of S-Larotrectinib;
b) the calculation by HPLC of the area corresponding to S-Larotrectinib in a sample of Larotrectinib sulfate;
c) the determination of the amount of S-Larotrectinib in a sample of Larotrectinib by comparing the area measured in step a) with that measured in step b).

The HPLC method used to determine the presence of S-Larotrectinib comprises:
a) the dissolution of the Larotrectinib sulphate sample to be analyzed in a solvent selected from water and acetonitrile or mixtures thereof;
b) the injection of the solution from step (a) into a reverse phase chromatographic column;
c) the elution of the solution of step (a) in a time comprised between 15-45 minutes, using a gradient of eluents A and B in different percentages;
d) the measurement of the S-Larotrectinib content in the Larotrectinib sulfate sample using a UV detector operating at a wavelength comprised between 190-280 nm.

The Larotrectinib sulfate sample is dissolved in a water / acetonitrile mixture in a variable ratio comprised between 9: 1 and 7: 3, advantageously in a ratio 8:2, so as to obtain a solution containing Larotrectinib sulfate at a concentration of 0.10-0.30 mg/ml, advantageously 0.20 mg/ml. The solution obtained in step a) is injected into a reverse-phase chromatographic column (octadecylsilane), preferably Waters X-Bridge C18 150x4.6 mm, 3.5 µm.

The sample is eluted in the column using a gradient of eluents A and B in different percentages, in a time comprised between 15-45 minutes, advantageously 20-40 minutes.

Eluent A consists of a solution of 0.63 g / L ammonium formate at pH 3 with formic acid, while eluent B is acetonitrile. The ratio between eluent A and B changes during the analysis, in particular between 0 and 1 minute the eluting mixture contains 90% phase A and 10% phase B, at 15 minutes 70% phase A and 30 % phase B, between 19 and 22 minutes 52% phase A and 48% phase B, between 27 and 32 minutes 25% phase A and 75% phase B, between 33 and 40 minutes 90% phase A and 10% phase B. The UV detector operates at a wavelength comprised between 190 and 280 nm, preferably 254 nm.

S-Larotrectinib is characterized by a relative retention time (RRT) comprised between 0.85-0.95, more particularly 0.87, in relation to Larotrectinib.

### S-Larotrectinib impurity determination

S-Larotrectinib impurity was isolated and characterized by HPLC, H-NMR and GC-MS.

### HPLC Analysis

UHPLC Thermo Fisher Vanquish system or equivalent equipped with: Autosampler with peltier system, UV Detector - Visible, Chromeleon data recording software or equivalent, column oven.
Column: Waters X-Bridge C18 150x4,6mm; 3.5 µm
Analysis time: 40 min
Acquisition time: 40 min
Injection volume: 5 µl
Flow: 1.0 ml /min
Column temperature: 35 °C
Auto sampler temperature: 5 °C
Wavelength: 260 nm
Diluent: H₂O/CH₃CN 8:2
Mobile phase A: 0.63 g/L of ammonium formate at pH 3 with formic acid
Mobile phase B: CH₃CN

Gradient elution as shown in the scheme below:

| Stage | Time (min) | Phase A(%) | Phase B(%) | Note |
|---|---|---|---|---|
| 1 | 1 | 90 | 10 | Isocratic |
| 2 | 15 | 70 | 30 | Linear gradient |
| 3 | 19 | 52 | 48 | Linear gradient |
| 4 | 22 | 52 | 48 | Isocratic |
| 5 | 27 | 25 | 75 | Linear gradient |
| 6 | 32 | 25 | 75 | Isocratic |
| 7 | 33 | 90 | 10 | Linear gradient |
| 8 | 40 | 90 | 10 | Conditioning |

### Selectivity of the method:

| COMPOUND | RT(min) | RRT |
|---|---|---|
| S-LAROTRECTINIB | 16.2 | 0.87 |
| LAROTRECTINIB SULFATE | 18.5 | 1.00 |

### Example 1

### Control of impurity formation - method reported in the application WQ2016077841

As proof of the formation of the S-Larotrectinib impurity, Larotrectinib sulfate was synthesized following the method described in WO2016077841 (example 2, page 66). The salification reaction with H₂SO₄ in EtOH, the solvent that solvates the crystal, necessarily causes a subsequent stress of the product which is refluxed in a mixture of water and methylethylketone.

The combination of time and temperature used to reach the specification for residual solvents in the active ingredient, required by the ICH Q3 A guideline (ethanol <5000 ppm), causes an increase in the S-Larotrectinib impurity.

Below is the table of monitoring data of the percentage of ethanol residual in the crystal and the amount of impurity formed over time during the stripping procedure of the crystal in H₂O: methylethylketone 5:95.

| Sample of Larotrectinib sulfate (batch 1693/72) | Stripping time | % EtOH Analysis ¹H-NMR | %S-Larotrectinib Analysis HPLC |
|---|---|---|---|
| | 0 minute | 0.95% | 0.04% |
| | 30 minutes | 0.30% | 0.06% |
| | 60 minutes | 0.30% | 0.08% |

### Example 2

### Synthesis of Larotrectinib sulfate containing less than 0.10% by weight of S-Larotrectinib

### a) Synthesis of Larotrectinib chloride

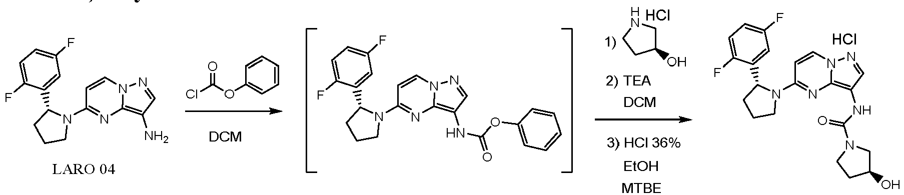

(R)-5-(2- (2,5-difluorophenyl) pyrrolidin-1-yl) pyrazole [1,5-a] pyrimidine-3-amine (LARO 04) (100 g, 0.317 mol) and dichloromethane (800 ml) are sequentially loaded into a reactor. The solution is left under stirring at 20°C for about 10 minutes.

A solution of phenylchloroformate (52.1 g, 0.329 mol) in dichloromethane (200 ml) is dropped, maintaining a temperature below 30°C and left under stirring at 28°C for about an hour.

3-(R)-hydroxy pyrrolidine HCl (47 g, 0.380 mol) is loaded into the reactor and triethylamine (112.3 g, 1.109 mol) is subsequently dropped, maintaining a temperature below 40°C. It is left under stirring at 40°C for about 4 hours.

A solution of citric acid (180 g, 0.936 mol) and demineralized water (420 ml) is prepared in another reactor and the previously prepared solution is loaded by dripping. The reaction is left under stirring for 15-20 minutes at room temperature, the phases are allowed to decant and the organic phase is separated from the aqueous one. The previously obtained organic phase is loaded into the flask. A solution of 35% HCl (900 g, 8.64 mol) in demineralized water (750 ml) is prepared in another reactor and cooled to a temperature between 5 and 10°C. Half of the 35% HCl solution maintained at room temperature is added to the organic phase. The mixture is left under stirring for 15-20 minutes, the phases are left to decant and the organic phase is separated from the aqueous one.

The other half of the 35% HCl solution at room temperature is added to the organic phase. The mixture is left under stirring for 15-20 minutes, the phases are left to decant and the organic phase is separated from the aqueous one.

The combined aqueous phases are loaded into the reactor and dichloromethane (1 L) is added. The mixture is left under stirring for 15-20 minutes, the phases are left to decant and the organic phase is separated from the aqueous one.

The washing step of the aqueous phase with dichloromethane is repeated for another 2 times.

A mixture composed of NaOH aq 30% (1430 g, 10.72 mol) and dichloromethane (1L) is prepared in another reactor and this mixture is dropped into the aqueous phase previously cooled to 5°C and 10 °C, maintaining the temperature below 30°C.

The mixture is left under stirring for about 30 minutes at 28°C, the phases are decanted and the organic phase is separated from the aqueous one. The organic phase is distilled under reduced pressure to a small volume.

The absolute ethanol (1200ml) is loaded onto residue and the organic phase is distilled under reduced pressure to small volumes. A solution of 17% HCl in ethanol (136 g, 0.634 mol) is slowly added to the reactor while maintaining the temperature at 28°C. The mixture is left under stirring at 28°C for about 2 hours. 700 ml of methyl-t-butyl ether (MTBE) are slowly added while maintaining the temperature at 28°C. The mixture is left stirred at 28°C for about an hour. It is filtered on buchner washing with a mixture of absolute ethanol (200 ml) and MTBE (400 ml). The solid thus obtained is dried under vacuum at 40°C for at least 6 hours. 116.0 g of pink-orange solid product (116%) are obtained.

### b) Synthesis of Larotrectinib sulfate

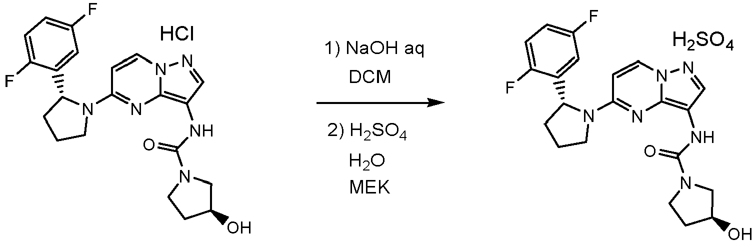

Larotrectinib chloride (100.00 g, 0.233 mol), dichloromethane (1 1) and demineralized water (300 ml) are sequentially loaded into a reactor. The suspension is stirred for 5 minutes at room temperature and then an aqueous solution of 30% NaOH (62.13 g, 0.466 mol) is dropped. It is left under stirring for 30 minutes at room temperature. The aqueous phase is separated from the organic one and the organic phase is distilled at reduced pressure. Methylethylketone (1 L) is loaded into the reactor and distilled under reduced pressure to a small volume. Demineralized water (50 ml) is added and the mixture is heated to 42°C. 40% H₂SO₄ is dropped for about 30 minutes maintaining the temperature comprised between 42 and 50°C. Methylethylketone (50 ml) is charged while maintaining the temperature below 50°C. The mixture is stirred for 15 minutes at 42°C. The mass is cooled to room temperature, methylethylketone (500 ml) is added and stirred at room temperature for 30 minutes.

The suspension is cooled to a temperature between 0°C and 5°C and is left stirred for at least one hour. It is filtered by washing the solid with methylethylketone (200 ml) and dried under vacuum at 28°C for at least 6 hours. 95.0 g of light pink solid (95%) are obtained.

### Comparative Example 3

In order to demonstrate the advantage in terms of purity on the final active ingredient, a preparation batch of Larotrectinib chloride (Example 2 stage a) was divided before the hydrochloride salt formation stage into three batches of equal weight.

The first batch was transformed into Larotrectinib chloride following the procedure reported in example 2, stage a), and subsequently the hydrochloride was transformed into Larotrectinib sulfate (batch: 1693/83-1), following the reaction reported in example 2, stage b).

The second batch was transformed directly into Larotrectinib sulfate (batch: 1693/83-2), following the procedure described in WO2016077841.

The third batch was transformed into Larotrectinib chloride following the procedure reported in example 2, stage a), and subsequently the hydrochloride was transformed into Larotrectinib sulfate (batch: 1693/72), following the reaction reported in example 2, stage b).

The three finished products analyzed show the following impurities:
batch: 1693/83-1: LARO 04 0.1% and S-Larotrectinib 0.08%
batch: 1693/83-2: LARO 04 0.35% and S-Larotrectinib 0.11%.
batch 1693/72: LARO 04 n.d. and S-Larotrectinib 0.04%

## Claims

1. A process for preparing Larotrectinib sulfate containing less than 0.10% by weight S-Larotrectinib, comprising:
(a) the unblocking reaction of Larotrectinib hydrochloride in dichloromethane, in the presence of bases selected from an alkaline or alkaline earth hydroxide;
(b) the treatment of Larotrectinib obtained in step a) with sulfuric acid in methyl ethyl ketone, and water;
(c) the filtration of Larotrectinib sulfate.

2. The process according to claim 1 wherein the base used in step (a) is selected from NaOH and KOH.

3. The process for the preparation according to claim 1 or 2 wherein step (b) is carried out by dropwise addition of a 40% by weight sulfuric acid aqueous solution into a solution of Larotrectinib in demineralized water and methyl ethyl ketone.

4. The process according to claim 3 wherein the ratio of demineralized water to methyl ethyl ketone is 5:95.

5. The compound (3S)-N-[5-[(2R)-2-(2,5-Difluorophenyl)-1-pyrrolidinyl]-pyrazole [1,5-a]pyrimidine-3-yl]-3-hydroxysulfonyloxy-1-pyrrolidinecarboxamide (S-Larotrectinib) of formula:

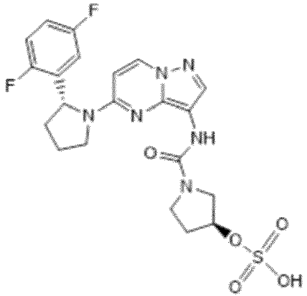

6. Use of S-Larotrectinib of claim 5 as analytical reference standard for the determination of purity of Larotrectinib sulfate.

7. A process for determining the presence of S-Larotrectinib in a Larotrectinib sulfate sample, comprising:
a) the determination, by HPLC analysis, of the retention time corresponding to S-Larotrectinib using a reference sample of S-Larotrectinib;
b) the determination, by HPLC analysis, of the retention time corresponding to S-Larotrectinib in a sample of Larotrectinib sulphate;
c) the determination of the presence of S-Larotrectinib in a sample of Larotrectinib sulphate by comparing the chromatograms obtained at steps a) and b).

8. A process for determining the amount of S-Larotrectinib in a Larotrectinib sulfate sample comprising:
a) the measurement, by HPLC, of the corresponding area S-Larotrectinib in the reference standard **characterized by** a known quantity of impurity S;
b) the measurement, by HPLC, of the area corresponding to S-Larotrectinib in a sample containing S-Larotrectinib and Larotrectinib sulfate and;
c) the determination of the amount of S-Larotrectinib in a sample of Larotrectinib sulfate by comparing the area measured at step a) with that measured at step b).

## Patentansprüche

1. Verfahren zur Vorbereitung von Larotrectinibsulfat, das weniger als 0,10 Gew.-% S-Larotrectinib enthält, umfassend:
(a) die Entblockungsreaktion von Larotrectinib-Hydrochlorid in Dichlormethan in Anwesenheit von Basen, die aus einem Alkali- oder Erdalkalihydroxid ausgewählt werden;
(b) Behandlung des in Schritt a) erhaltenen Larotrectinibs mit Schwefelsäure in Methylethylketon und Wasser;
(c) die Filtration von Larotrectinibsulfat.

2. Verfahren nach Anspruch 1, wobei die in Schritt (a) verwendete Base aus NaOH und KOH ausgewählt wird.

3. Verfahren zur Vorbereitung nach Anspruch 1 oder 2, wobei der Schritt (b) mittels tropfenweiser Zugabe einer 40 Gew.% wässrigen Schwefelsäurelösung in eine Lösung von Larotrectinib in entmineralisiertem Wasser und Methylethylketon durchgeführt wird.

4. Verfahren nach Anspruch 3, wobei das Verhältnis von demineralisiertem Wasser zu Methylethylketon 5:95 beträgt.

5. Verbindung (3S)-N-[5-[(2R)-2-(2,5-Difluorphenyl)-1-pyrrolidinyl]-pyrazol[1,5-a]pyrimidin-3-yl]-3-hydroxysulfonyloxy-1-pyrrolidincarboxamid (S-Larotrectinib) der Formel:

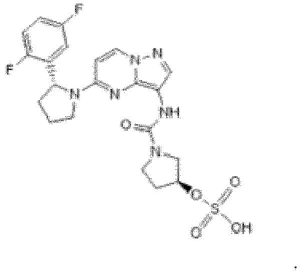

6. Verwendung von S-Larotrectinib nach Anspruch 5 als analytischer Referenzstandard zur Bestimmung der Reinheit von Larotrectinibsulfat.

7. Verfahren zur Bestimmung der Anwesenheit von S-Larotrectinib in einer Larotrectinibsulfatprobe, umfassend:
a) die Bestimmung, mittels HPLC-Analyse, der S-Larotrectinib entsprechenden Retentionszeit unter Verwendung einer Referenzprobe von S-Larotrectinib;
b) die Bestimmung, mittels HPLC-Analyse, der S-Larotrectinib entsprechenden Retentionszeit in einer Probe von Larotrectinibsulfat;
c) die Bestimmung der Anwesenheit von S-Larotrectinib in einer Probe von Larotrectinibsulfat durch das Vergleichen der in den Schritten a) und b) erhaltenen Chromatogramme.

8. Verfahren zur Bestimmung der Menge von S-Larotrectinib in einer Larotrectinibsulfatprobe, umfassend:
a) die Messung, mittels HPLC, der entsprechenden Fläche S-Larotrectinib in dem Referenzstandard, der durch eine bekannte Menge an Verunreinigung S gekennzeichnet ist;
b) die Messung, mittels HPLC, der S-Larotrectinib entsprechenden Fläche in einer Probe, die S-Larotrectinib und Larotrectinibsulfat enthält;
c) die Bestimmung der Menge von S-Larotrectinib in einer Probe von Larotrectinibsulfat durch das Vergleichen der in Schritt a) gemessenen Fläche mit der in Schritt b) gemessenen Fläche.

## Revendications

1. Procédé de préparation de sulfate de Larotrectinib contenant moins de 0,10 % en poids de S-Larotrectinib, comprenant :
(a) la réaction de déblocage du chlorhydrate de Larotrectinib dans le dichlorométhane, en présence de bases choisies parmi les hydroxydes alcalins ou alcalino-terreux ;
(b) le traitement du Larotrectinib obtenu à l'étape a) avec de l'acide sulfurique dans de la méthyléthylcétone et de l'eau ;
(c) la filtration du sulfate de Larotrectinib.

2. Procédé selon la revendication 1, dans lequel la base utilisée à l'étape (a) est choisie parmi NaOH et KOH.

3. Procédé de préparation selon la revendication 1 ou 2, dans lequel l'étape (b) est réalisée par addition goutte à goutte d'une solution aqueuse d'acide sulfurique à 40 % en poids dans une solution de Larotrectinib dans de l'eau déminéralisée et de la méthyléthylcétone.

4. Procédé selon la revendication 3, dans lequel le rapport entre l'eau déminéralisée et la méthyléthylcétone est de 5:95.

5. Composé (3S)-N-[5-[(2R)-2-(2,5-Difluorophényl)-1-pyrrolidinyl]-pyrazole [1,5-a]pyrimidine-3-yl]-3-hydroxysulfonyloxy-1-pyrrolidinecarboxamide (S-Larotrectinib) de formule :

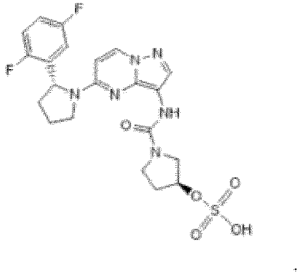

6. Utilisation du S-Larotrectinib selon la revendication 5 comme norme de référence analytique pour la détermination de la pureté du sulfate de Larotrectinib.

7. Procédé de détermination de la présence de S-Larotrectinib dans un échantillon de sulfate de Larotrectinib, comprenant :
a) la détermination, par analyse HPLC, du temps de rétention correspondant au S-Larotrectinib à l'aide d'un échantillon de référence de S-Larotrectinib ;
b) la détermination, par analyse HPLC, du temps de rétention correspondant au S-Larotrectinib dans un échantillon de sulfate de Larotrectinib ;
c) la détermination de la présence de S-Larotrectinib dans un échantillon de sulfate de Larotrectinib en comparant les chromatogrammes obtenus aux étapes a) et b) .

8. Procédé de détermination de la quantité de S-Larotrectinib dans un échantillon de sulfate de Larotrectinib comprenant :
a) la mesure, par HPLC, de la surface correspondante de S-Larotrectinib dans la norme de référence **caractérisée par** une quantité connue d'impureté S ;
b) la mesure, par HPLC, de la surface correspondant au S-Larotrectinib dans un échantillon contenant du S-Larotrectinib et du sulfate de Larotrectinib et ;
c) la détermination de la quantité de S-Larotrectinib dans un échantillon de sulfate de Larotrectinib en comparant la surface mesurée à l'étape a) avec celle mesurée à l'étape b).
